# EUROPEAN PATENT APPLICATION

(11) **EP 0 550 008 A2**
(43) Date of publication of application: **07.07.1993**
(21) Application number: 92121864.0
(22) Date of filing: 23.12.1992
(51) Int. Cl.: A61K 31/16

(54) **Use of N-acyl derivatives of aminoalcohols with polycarboxylic acids for the manufacture of a medicament for the treatment of pathologies relating to mast cells**

(30) Priority: 31.12.1991 IT MI913509
(71) Applicant: LIFEGROUP S.p.A., I-35043 Monselice (Padova) (IT)
(72) Inventor: Della Valle, Francesco, I-35122 Padova (IT); Lorenzi, Silvana, I-35142 Padova (IT); Marcolongo, Gabriele, I-35020 Carrara San Giorgia (Padova) (IT)
(74) Representative: Gervasi, Gemma, Dr.

(57) **Abstract**

N-acyl derivatives of aminoalcohols with polycarboxylic acids able to modulate the degranulation process consequent to the mast cells activation in inflammatory processes caused by overmaximal stimuli of neurogenic and immunogenic origin.

## Description

### FIELD OF THE INVENTION

The present invention relates to the use of N-acyl derivatives of aminoalcohols with polycarboxylic acids for the preparation of pharmaceutical compositions for the therapeutic treatment of pathologies undergoing mast cells degranulation, consequent to a neuroimmunogenic and/or immunogenic hyperstimulation.

### PRIOR ART DISCLOSURE

It is known that, when the local homeostasis at the level of the individual systems or compartments is disturbed as a consequence of an exogenous or an endogenous noxa, the human organism uses a complex biologic defence system consisting of a debris removal and of reparative remodeling, said system being known as the inflammatory process.

This process involves locally sequential changes finely regulated by vascular homeostasis, massive cellular infiltrations, local release of several specific chemical mediators and a proliferous and reparative response induction, directed to restore the regular homeostasis in the damaged zone.

This defence and restoration physiological process may assume pathologic features under the conditions of chronic persistence of the noxa initial signals, for example in the autoimmune processes or in case of lack of regulation and control of the physiological process development, as it may occur in the processes of reactive hypersensitivity.

In the autoimmune pathologies the persistence of the trigger noxa, results very clearly, as it is represented by the autoantigene, which is of course present in the organism and it is by mistake identified by the immune system as exogenous noxa to be destroyed.

A very important and up to now not very investigated role is played in the physiologic inflammatory processes by a particular cellular population residing in the tissues, known as Mast cells or mast cells. These cells begin the inflammatory process, after their activation thanks to specific signals, achieved through the massive release of several mediators being locally active on the vascular bed, the recall, the activation and the migration from the vasal to the tissue compartment of the cellular populations involved in the inflammatory and reparative process. Because of this crucial role the mast cell population is nowadays aknowledged as the effector system of the inflammation.

The mast cell is therefore at the center of complex interactions, which have an ascertained both physiological and pathophysiological meaning among the nervous system, the endocrinous system and immune system and may be summarized as:
a) pro-inflammatory agonism coming from the nervous system through the neuropeptides, in particular the substance P (neurogenic inflammation);
b) pro-inflammatory agonism coming from the immune system through IgE and Interleukin 1 (immunophlogosis);
c) trophic effects of nervous origin (NGF);
d) negative feed back of the control of the endocrinous system mediated by the corticosteroid hormones. (J. Olsson, Int. Rev. Citol., 1968,24, P.27-70; Marshall J.S.and Bienenstock J. Springer, Semin.Immunopath. 1990,12, 191-202).

The mast cell represents at the cellular level the link between the nervous system and the immune system, being its activation under physiological and decidedly pathologic conditions, strictly controlled by both the nervous and the immune system.

Under neuroimmunogenic hyperstimulation conditions, which are present and aggravating in many pathologies having a strong inflammatory component, the mast cell realizes through a degranulation process its "aggressive" potential against its target organ, by releasing cytolesive substances.

In order to understand completely its pathophysiological meaning, it is important to note that mast cells are widely present in different systems and organs and that, according their tissue subdivision, are distinguishable in two different classes.

In fact both connective and muconasal mast cells were identified.

These two mast cells population, although possessing different histochemical and biochemical properties, are both characterized by the ability to synthetize a great variety of cytokins, as well as conventional mediators such as histamine, serotonin , lipidic products, as for example arakidonic acid and prostaglandins and also polysaccharides, such as the heparins and to preserve them in specific stores, which they are released from, as a consequence of the above mentioned neuroimmunogenic stimulation.

As far as the cytokins are concerned the presence of the Tumor Necrosis Factor (TNF) in preformed prompt release secretory granules results to be of particular relevance (Gordon et al., 1990, Nature, 346 (6281),274-276).

Cytolithic and antitumoral properties are attributed to TNF, properties which are generally realized through cytotoxicity processes, but also through articular inflammation processes, proliferation of dermic fibroblasts, osseous remodeling, stimulation of the toxicity of eusinophils in parasitogenic infections.

One of the most interesting aspects resides in the synthesis conditions of this cytokin, since, as it was previously affirmed, it is also present under cellular rest conditions in the form of granular storages, under a neuroimmunogenic type stimulus the mast cells release immediately the preformed TNF, and they synthetize it ex novo.

TNF is therefore the mast cell mediator, which is immediately released, showing then a kinetics extended in time, unlike other prompt release mediators, such as histamine, and that is important in the so called "late phase reactions" which are the main cause of the tissue damage of pathologies associated with mast cells degranulation.

Close to a considerable interest for the activation mechanisms of mast cells, an analogous attention was not observed in the research of the endogenous route of modulation and control of the mast cell hyperactivity, which may be found under many pathological conditions.

All the complex biological systems are regulated by countered mechanisms of agonism and antagonism.

To the neuromediated or immunomediated sequences of mast cell activity, having an agonist meaning towards degranulation, inhibitory antagonist mechanisms acting through local and /or general circuits must correspond in order to maintain the homeostatic equilibrium.

An endogenous mechanism of systemic control of the mast cells behaviour is in any case known, since the endogenous corticosteroid hormones exhibit an antidegranulating activity, preferential for the degranulation induced by immunologic route.

(Bergstrand E.,Bjornsson A., Lundquist B.,Nilsson A. and Brattstand R. Allergy, 39, p. 217-230, 1984).

These hormones are in any case endowed with pleiotropic activity at the level of many systemic targets, and they cannot be considered specific antagonists of the mast cell activity, but rather they must be considered as a macro-response of defence, in case all the local systems of control are overcome by massive or generalized insults.

A local control system, acting through endogenous substances representing a first defensive barrier against the pathologic event is however less known.

From a therapeutic point of view up to now all the attention has been exclusively concentrated on the mast cell as a target for exogenous drugs able to inhibit the histamine release as the first and most important mediator involved in the prompt responses of the activation of the inflammatory processes.

It is of the early fifties the quite casual discovery of an antiinflammatory activity in a lipidic excipient, utilized as the vehicle of an antirheumatic drug (Long D.A. and Miles A.A., Lancet, 1950, p.492).

The successive research directed to identify the agent able to show this action evidenced its presence both in vegetable materials (peanuts oil, soya seeds), and in animal materials (yolk egg mainly).

Only in the middle fifties Kuehl et al.(Kuehl F.A., Jacob T.A., Ganley O.H., Ormond R.E. and Meisinger M.A.P. J. American Chemical Society , 1957, 79 (19), p 5577/8) were able to isolate this substance and then to define its chemical structure which was confirmed to be of N-palmitoylethanolamine (N-PEA), also in view of the comparisons made with the same product obtained by synthesis. (Ganley O.H., Groesslet O.E. and Robinson H.J., J.Lab. and Clin. Med., 1958, 51(5), p.709-714).

The successive pharmacological characterization indicated that N-PEA was active in many inflammation models ( Masek K. and Raskova H.,Int. Symp. on "Drugs of animal origin" 1966, Ferri Edizioni, p. 166-209) and furthermore it resulted also active in increasing the resistance of the animals against various bacterial toxins, and experimental infections.

For the pharmaceutical development, which was successively carried out by these Czehoslovak researchers the immunostimulating effect of resistance against the bacterial toxins and the experimental infections was considered more important.

A pharmaceutical formulation was prepared and was available on the Czechoslovak market in tablets, each containing 300 mg of active principle known with the name IMPULSIN, and it had, as the main therapeutic indication, the prevention of infections in the respiratory tract.

After few years this product was retired from the market because of the poor interest for the chosen indication.

Along the lines followed by the first studies on the identification of these compounds both in vegetable and in animal tissue, an active biochemical type research continued, which was directed to isolate and characterize particular classes of lipids deriving from the N-acylation of membrane phospholipids. In particular specific classes of phospholipds resulted to be abundant in vegetables tissue and specifically in soybean and pea (Aneja R., Chadha J.S. and Knaggs J.A., Biochem. Biophys. Res. Comm. 36 (3), p. 401-406, 1969; Dawson R.M.C., Clarke N. and Quarles R.H., Biochem. J. 1969,114 p.265-270).

The identification of these compounds in germinal cellular layers or in particular differentiation and/or degeneration states (Gray G.M., Biochim. Biophys. Acta, 1976, 431, p. 1-8; Somerharju P. and Renkonen P., Biochim. Biophys. Acta, 1979, 573, p.83-89) let hypothyze the existence of a specialized functional role of these compounds, although the hypothesis was initially put forth that these products were the result of the activation of the catabolic routes, and therefore they were degradation products.

The possibility that these compounds could represent a physiological form of defence, directed to block and /or to minimize the damage induced by hypotoxic stress was more clearly put forward by Epps et al. ( Biochem. Biophys. Res. Comm., 1979, 90 (2), p.628-633), who found elevated concentrations of N-acylethanolamine in myocardium areas affected with infarction in the dog after coronaric ligature.

It was supposed by these authors that these compounds had a physiologic meaning of defence, just in relation to the antiinflammatory activities of N-PEA previously found, without, however, indicating a possible mechanism and /or a preferential site of action, which could correlate the two experimental observations, namely their accumulation in hypotoxic areas , and their antiinflammatory activity .

### DISCLOSURE OF THE INVENTION

The Applicant has now found compounds able to modulate the degranulation process consequent to the mast cells activation in inflammatory processes caused by overmaximal stimuli of neurogenic and immunogenic origin.

These compounds are the N-acyl derivatives of aminoalcohols, preferably of ethanolamine, diethanolamine, (2-hydroxy-propyl)-amine, di-(2-hydroxy-propyl)-amine, wherein the acyl group derives from bicarboxylic and tricarboxylic acids.

The present invention therefore relates to the use of these N-acyl derivatives for the preparation of pharmaceutical compositions for the therapeutic treatment of pathologies undergoing to mast cells degranulation, as a consequence of a neurogenic and/or immunogenic hyperstimulation.

### DETAILED DESCRIPTION OF THE INVENTION

The characteristics and advantages of the N-acyl derivatives of the aminoalcohols suitable for the treatment of the pathologies subjected to mast cell degranulation according to the present invention, will be better illustrated during the course of the present detailed description.

The applicant has in fact found that the compounds having these pharmacological properties both in human beings and animals are the N-acyl derivatives of aminoalcohols having the formula: 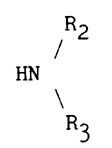
wherein R₂ is an alcoholic residue selected from a C₁-C₂₀ linear or branched hydroxyalkyl optionally substituted in the aliphatic chain with one or more aryl groups and a hydroxyaryl optionally substituted with one or more linear or branched alkyl radicals of from 1 to 20 carbon atoms, and R₃ is H or is = R₂.

The preferred aminoalcohols are monoethanolamine, diethanolamine, 2-hydroxypropylamine, di-(2-hydroxy-propylamine), whereas the acyl group belongs to a polycarboxylic acid.

In case the aminoalcohols is 2-hydroxypropylamine or di-(2-hydroxy) propylamine, the corresponding N-acylderivative is a raceme or an optical isomer. Examples of the above mentioned compounds are represented by the following formulas: 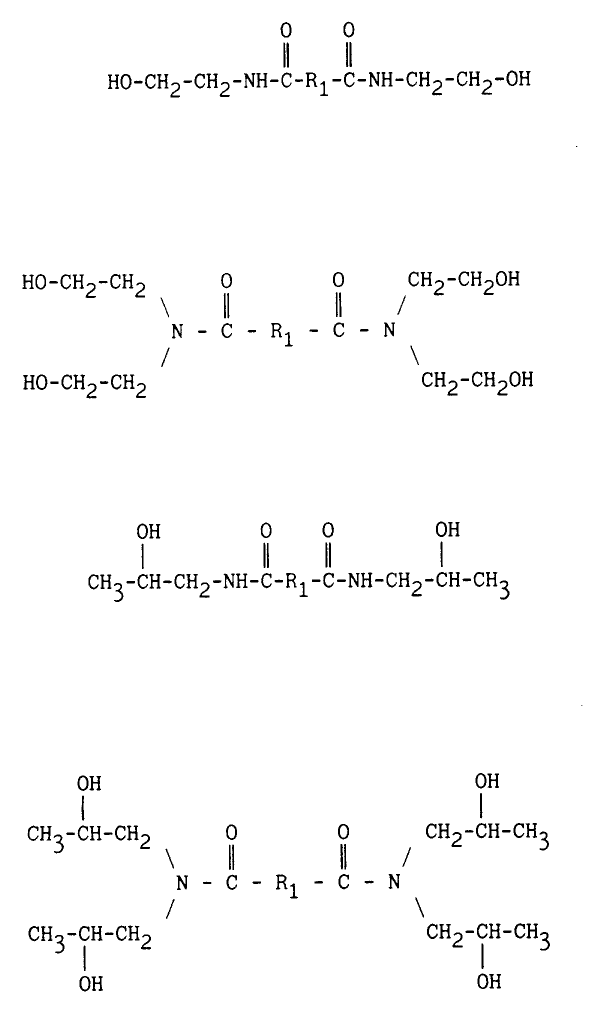
wherein 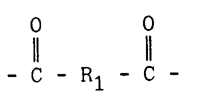
is the acyl radical of a saturated or an unsaturated aliphatic dicarboxylic acid, optionally substituted with a hydroxy or an aminic group, or of an aromatic, heterocyclic or heteroaromatic dicarboxylic acid.

For merely illustrative purposes among the saturated or unsaturated aliphatic dicarboxylic acids there are to be cited oxalic, fumaric, azelaic, succinic, traumatic , glutaric acid, the muconic acids, particularly the trans, trans isomer of cromoglycholic acid, and the hydroxy- or amino- substituted homologues of all these acids, and particularly : malic, tartaric, aspartic, glutammic.

Among the aromatic, heterocyclic and heteroaromatic dicarboxylic acids there are to be cited, phthalic, 1,4 dihydroxy-2,6-dimethyl-4-(2-nitrophenyl)-3,5-pyridin-dicarboxylic acid, 4,6-dioxo-1-ethyl-10-propyl-4H,6H,-pyran-[3,2g]-quinolin-2,8-dicarboxylic, cromoglycholic acid and other biologically acceptable salts.

Among the tricarboxylic acids particularly preferred is the citric acid.

As already observed, the N-acyl derivatives of the aminoalcohols according to the present invention find their applications in all the human and animal pathologies characterized by an inflammatory state having neuroimmunogenic also on autoimmune base, in which it is necessary to carry out a local and selective modulation of the mast cell.

The modulation obtained by a controlled inhibition of the maximal degranulation process of the mast cells activated by neuroimmunogenic stimulations, acting by pharmacological route with the substances according to the present invention, results in fact to be an important therapeutical instrument in all the pathologies wherein the mast cell represents the aetiopathogenetic effector system.

Taking into account for illustrative purposes the autoimmune field, the most recent prior art provides therapeutically important implications, since the mast cells result to be pathophysiologically involved in many pathologies as for example the most famous multiple sclerosis and psoriasis.

In multiple sclerosis the pathologically activated mast cells seem to be the cause of plaques production in CNS, because the hematoencephalic barrier is compromised, because of the adhesion of the infiltrating granulocytes from the vasal compartment and finally because of the release of the aggressive cytokin Tumour Necrosis Factor, the final aggressor of the myelinic structures and the last cause of the histic damages. (Toms R.et al., J. Immunology , 1990, p.169-177; Kruger P.G. et al., Acta Neurol. Scand., 1990,81, p.31-36).

Also in the case of psoriasis the chronic localized inflammation seem to be directly correlated to vicious mast cells activation.

As a matter of fact mast cells degranulation intervenes precociously in the psoriatic lesions development whereas in the chronic situations a considerable increase in mast cells, present in the lesions, is observed (Toyry S. et al., Arch. Dermatol. Res.,1988,280,p.282-285; Turiniopwa B. and Jablonka S., Arch. Dermat. Res. 1988,280, p.189-193).

These two pathologies are not the only ones which can be advantageously treated with the aminoalcohols N-acyl derivatives according to the present invention, since they are not the only pathologies wherein a mast cell activation is the precocious etiopathogentic effector system of the autoaggressive processes, whose first symptom, locally developing, is an inflammatory process. In fact the local activation of the mast cells, already residing in the tissues, precedes and conditions the local infiltration of typical cellular populations of inflammation.

The mast cells degranulation seems to determine the vasal permeability, and the endothelial adhesion of the leukocytes, which are recruited by the circle to act on the spot of the pathologic insult. This mechanism has been recently proposed for psoriasis by Matis et al., on the base of precise experimental data (Matis et al.,J. Inv . Dermatol. 94,1990, p.492-495).

All the compounds of the above defined class may therefore find a valid therapeutic application, according to their own specific pharmacologic activity in all the other dermatologic pathologies having autoimmune origin such as atopic dermatitis, dermatomyositis, scleroderma, polymyositis, pemphigus, pemphigoid, epidermolysis bullosa, or ophtalmic pathologies as for example Sjogren's syndrome, sympathetic ophtalmia, autoimmune uveitis and uveoretinites or again in articular and connective pathologies such as rheumatoid arthritis, psoriatic arthritis, systemic lupus erythematosus arthritis, systemic and discoid lupus erythematosus.

Other conditions wherein it is therapeutically useful the local control of the mast cells degranulation process are the chronic inflammatory pathologies, having autoimmune origin as for example the chronic inflammations of the gastrointestinal mucous membranes (Crohn's disease).

In addition the compounds according to the present invention are effective in the treatment of other pathologies having immunologic origin where the mast cells hyperactivation is observed.

In this regard we may say that the numerical increase and the increase in functional activation of the mast cells is observed in all the allergic and inflammatory phenomena and in many pathologic situations associated with fibrosis.

Besides the already mentioned pathologies having autoimmune origin we may also cite the following ones: among the chronic inflammatory pathologies chronic heliumdermatitis, interstitial pulmonary fibrosis, among the allergic pathologies the allergic conjunctivitis and giant papillary conjunctivitis, dietetic allergies; among the cicatrization disorders hypertrophic and keloid scars.

Furthermore, with regard to the animal pathology, the local anti-inflammatory effect exerted by these new derivatives is useful in the therapy of neurogenic inflammation (i.e. intravertebral disk disease), articular or connective pathologies, respiratory pathologies, laminitis (in which the use of corticosteroids is absolutely banned), eye pathology i.e. Keratoconjunctivitis sicca and finally inflammatory allergic manifestation, including food allergy.

We report herewith for illustrative but not limitative purposes the following examples of the preparation of N-acyl derivatives according to the present invention.

### Example 1 preparation of N-fumaroylethanolamide (1)

1.1 ml fumaroylchloride in 20 ml diethyl ether were added drop by drop to 3.6 ml ethanolamine dissolved in 50 ml methanol and 100 ml diethyl ether in 30 minutes while maintaining the reaction temperature at 0 °C.

The reaction was carried out for 1 hour at 0 °C and then completed at room temperature in 4-5 hours.

The obtained precipitate was dissolved in methanol and crystallized in diethyl ether.

The reaction yield was about 70%.

The physical-chemical characteristic of the N-fumaroyl ethanolamide product synthetized according to the example 1, are the following:

| | |
|---|---|
| - physical state | white crystalline powder |
| - raw formula | C₈H₁₄N₂O₄ |
| - molecular weight | 202.2 |
| - elemental analysis | C=47.52%; H=6.98%; N=13.85% 0=31.65% |
| - solubility in organic solvents | hot ethanol < 20 mg/ml DMSO < 20 mg/ml |
| - solubility in water | < 20 mg/ml |
| - melting point | 239 °C decomp. |
| - TLC | chloroform / methanol / H₂O (80 : 20 : 2) Rf = 0.34 |

### Example 2 preparation of N,N,N',N'- tetra (2-hydroxyethyl)-fumaroyl-diamide (2)

1.53 g fumaroyl chloride (10 mmol) in 20 ml anhydrous diethyl ether were slowly added drop by drop in 30 minutes to a solution of 4.4 g diethanolamine (42 mmol) in 50 ml anhydrous methanol and 100 ml diethyl ether at 0 °C under continuous stirring.

The obtained mixture was maintained at 0 °C for 1 hour under stirring and then at room temperature for 5 hours.

The obtained suspension was filtered, the filtrate was disregarded and the precipitate was crystallized from 20 ml ethanol / isopropanol 1:1, the product was separated by filtration, washed 3 times with 5 ml cool isopropanol and finally dried under high vacuum.

The reaction yield was about 78 %.

The physical chemical characteristic of N,N,N',N'-tetra (2-hydroxyethyl)-fumaroyl diamide are the following:

| | |
|---|---|
| - physical state | white crystalline powder |
| - raw formula | C₁₂H₂₂N₂O₆ |
| - molecular weight | 290.32 |
| - elemental analysis | C=49.65%; H=7.64%; N=9.65%; O=33.07%. |
| - solubility in organic solvents | > 10 mg/ml in DMSO; |
| | > 10 mg/ml in hot ethanol. |
| - solubility in water | > 10 mg/ml |
| - melting point | 128 - 130 °C |
| - TLC | chloroform/methanol/H₂O/NH₃ (28%) (80 : 25 : 2 : 1) Rf = 0.24 |

### Example 3 preparation of N,N'-bis-(2-hydroxyethyl)-nonandiamide (3)

A mixture of 1.88 g azelaic acid (10 mmol) and 1.84 g ethanolamine (30 mmol) was introduced into a flask fitted with a reflux condenser and heated to 160 °C for 6 hours in an oil bath.

The reaction mixture was directly crystallized from 50 ml isopropanol, the crystallized product was separated by filtration, washed 3 times with 10 ml cool isopropanol and finally dried under high vacuum.

The reaction yield was about 78 %.

The physical-chemical characteristics of the N,N'-bis (2-hydroxyethyl)-nonandiamide product are the following:

| | |
|---|---|
| - physical state | white crystalline powder |
| - raw formula | C₁₃H₂₆N₂O₄ |
| - molecular weight | 274.37 |
| - elemental analysis | C=56.91%; H=9.55%; N=10.21%; O=23.3%. |
| - solubility in organic solvents | > 10 mg/ml |
| - melting point | 132 - 134 °C |
| - TLC | chloroform/methanol/H₂O/NH₃ (28%) (80 : 25 : 2 : 1) Rf = 0.48. |

### Example 4 preparation of N,N'-bis-(2-hydroxyethyl)-succinamide (4)

A mixture of 1.18 g succinic acid (10 mmol) and 1.84 g ethanolamine (30 mmol) was introduced into a flask fitted with a reflux condenser and heated to 160 °C for 6 hours in an oil bath.

The reaction mixture was directly crystallized from 20 ml isopropanol, the crystallized product was separated by filtration, washed 3 times with 5 ml cool isopropanol and finally dried under high vacuum.

The reaction yield was about 75 %.

The physical-chemical characteristics of the N,N'-bis (2-hydroxyethyl)-succinamide are the following:

| | |
|---|---|
| - physical state | white crystalline powder |
| - raw formula | C₈H₁₆N₂O₄ |
| - molecular weight | 204.2 |
| - elemental analysis | C=47.06%; H=7.90%; N=13.72%; O=31.34%. |
| - solubility in organic solvents | > 10 mg/ml in DMSO |
| - melting point | 153 - 155 °C |
| - TLC | chloroform/methanol/H₂O/NH₃ (28%) (80 : 25 : 2 : 1) Rf = 0.24. |

### Example 5 preparation of N,N'-bis(2-hydroxyethyl)-oxalyldiamide (5)

1.46 g diethyloxalate (10 mmol) were slowly added drop by drop in 30 minutes to 1.34 g ethanolamine (22 mmol) at 0 °C under stirring. The obtained mixture was maintained at 0 °C for 1 hour and then at room temperature for 5 hours. The raw product thus obtained was directly crystallized from 50 ml 80% ethanol, the crystallized product was separated by filtration, washed three times with 10 ml cool ethanol and then dried under high vacuum.

The reaction yield was about 91 %. The physical-chemical characteristics of the N,N'-bis (2-hydroxyethyl)-oxalyldiamide are the following:

| | |
|---|---|
| - physical state | white crystalline powder |
| - raw formula | C₆H₁₂N₂O₄ |
| - molecular weight | 176.18 |
| - elemental analysis | C=40.9%; H=6.87%; N=15.90%; O=36.33%. |
| - solubility in organic solvents | > 10 mg/ml in DMSO |
| - solubility in water | > 10 mg/ml |
| - melting point | 168.5 - 170.5 °C |
| - TLC | chloroform/methanol/H₂O/NH₃ (28%) (80 : 25 : 2 : 1) Rf = 0.43. |

### Example 6 preparation of N,N'-bis (2-hydroxyethyl)trans-2-dodecendiamide (6)

A solution of 5.74 g isobutylchloroformiate (42 mmol) in 50 ml THF was slowly added drop by drop in 30 minutes to a mixture containing 4.57 g traumatic acid (20 mmol) and 4.26 g triethylamine (42 mmol) in 150 ml anhydrous THF under stirring at -10 °C.

The mixture was maintained under stirring at -10 °C for 2 hours and subsequently at 0 °C for 15 hours. 3.5 g ethanolamine were slowly added drop by drop in 30 minutes.

After the reaction was left under stirring for further 6 hours at 0 °C, the suspension thus obtained was filtered, the filtrate was disregarded and the precipitate was dried under vacuum. The raw product thus obtained was crystallized from 100 ml water, the product was filtered, washed 3 times with 20 ml water and finally dried under high vacuum.

The reaction yield was about 78 %.

The physical-chemical characteristics of the N,N'-bis (2-idroxyethyl)-dodecendiamide are the following.

| | |
|---|---|
| - physical state | white crystalline powder |
| - raw formula | C₁₆H₃₀N₂O₄ |
| - molecular weight | 314.43 |
| - elemental analysis | C=61.12%; H=9.62%; N=8.91%; O=20.35%. |
| - solubility in organic solvents | > 10 mg/ml in DMSO; >10 mg/ml in ethanol. |
| - solubility in water | > 10 mg/ml at 95 °C |
| - melting point | 134 - 136 °C |
| - TLC | chloroform/methanol/H₂O/NH₃ (28%). (80 : 25 : 2 : 1) Rf=0.57. |

### Example 7 preparation of N,N' -(2-hydroxyethyl)-malondiamide (7)

A mixture of 1.32 g dimethylmalonate (10 mmol) and 1.34 g ethanolamine (22 mmol) was introduced into a flask fitted with a reflux condenser and heated to 50°C for 4 hours in an oil bath.

The reaction mixture was directly crystallized from 20 ml isopropanol, the crystallized product was separated by filtration, washed 3 times with 10 ml of cool isopropanol and finally dried under high vacuum.

The reaction yield was about 92 %.

The physical-chemical characteristics of the N,N'-bis (2-hydroxyethyl)-malondiamide are the following:

| | |
|---|---|
| - physical state | white crystalline powder |
| - raw formula | C₇H₁₄N₂O₄ |
| - molecular weight | 190.2 |
| - elemental analysis | C=44.21%; H=7.42%; N=14.73%; O=33.65%. |
| -solubility in organic solvents | > 5 mg/ml in DMSO; >10 mg/ml in ethanol. |
| - solubility in water | > 10 mg/ml |
| - melting point | 127 - 129 °C |
| - TLC | chloroform/methanol/H₂O/NH₃ (28%) (80 : 25 : 2 : 1) Rf = 0.38. |

### Example 8 preparation of N,N'-bis (2-hydroxyethyl)-(+/-)2-hydroxysuccinimide (8).

2 g anhydrous sulfonic resin Dowex 50 x 8 H+ were added to 1.34 g DL malic acid (10 mmol) dissolved in 20 ml anhydrous methanol; the mixture was maintained at 30 °C under stirring for 20 hours. The resin was separated by filtration, the solution was evaporated to dryness and the residue was treated with 1.34 g of ethanolamine for 20 hours at 30 °C. The reaction mixture was directly crystallized from 50 ml isopropanol, the crystallized product was separated by filtration, washed 3 times with 10 ml of cool isopropanol and finally dried under high vacuum. The reaction yield was about 73 %.

The physical-chemical characteristics of the N,N'-bis (2-hydroxyethyl)-(+/-)-2-hydroxysuccinimide are the following:

| | |
|---|---|
| - physical state | white crystalline powder |
| - raw formula | C₈H₁₆N₂O₅ |
| - molecular weight | 220.23 |
| - elemental analysis | C=43.63%; H=7.32%; N=12.72%; O=36.33%. |
| - solubility in organic solvents | > 10 mg/ml in DMSO |
| - solubility in water | > 10 mg/ml |
| - melting point | 124 - 125 °C |
| - TLC | chloroform/methanol/H₂O/NH₃ (28%) (88 : 25 : 2 : 1) Rf = 0.17. |

### Example 9 preparation of (R,R)-(+) 2,3-dihydroxy-N,N'-bis (2-hydroxyethyl)-succinamide (9).

A mixture of 1.78 g dimethyl-L-tartrate (10 mmol) and 1.34 g ethanolamine (22 mmol) was introduced into a flask fitted with a reflux condenser and heated to 40 °C for 20 hours .

The reaction mixture was directly crystallized from 50 ml ethanol, the crystallized product was separated by filtration, washed 3 times with 10 cool ethanol and finally dried under high vacuum.

The reaction yield was about 90 %.

The physical-chemical characteristics of the (R,R)-(+) 2,3-dihydroxy-N,N'-bis(2-hydroxyethyl)-succinamide are the following:

| | |
|---|---|
| - physical state | white crystalline powder |
| - raw formula | C₈H₁₆N₂O₆ |
| - molecular weight | 236.25 |
| - elemental analysis | C=40.67%; H=6.83%; N=11.86%; O=40.64%. |
| - solubility in organic solvents | > 10 mg/ml in DMSO; 10 mg/ml in methanol. |
| - solubility in water | > 10 mg/ml |
| - melting point | 143 - 145 °C |
| - TLC eluent | chloroform/methanol/H₂O/NH₃ (28%) (88 : 25 : 2 : 1) Rf = 0.14. |

### Example 10 preparation of N,N'-bis (2-hydroxypropyl)-fumaroyldiamide (10).

1.53 g fumaroylchloride (10 mmol) dissolved in 20 ml anhydrous diethyl ether were slowly added drop by drop to a solution of 3.16 g 3-amino-2-propanol (42 mmol) in 50 ml anhydrous methanol and 100 ml anhydrous diethyl ether in 30 minutes while maintaining the reaction temperature at 0 °C.

The reaction was carried out for 1 hour at 0 °C, and completed at room temperature in 5 hours and the reaction mixture was finally evaporated to dryness.

The obtained residue was solubilized in 100 ml water and deionized with 30 ml of Bio-Rad AG 501 x 8 resin and finally lyophilized.

The reaction yield was about 75%.

The physical-chemical characteristics of the N-N'-bis (2-hydroxypropyl)-fumaroyldiamide are the following:

| | |
|---|---|
| - physical state | white amorphous powder |
| - raw formula | C₁₀H₁₈N₂O₄ |
| - molecular weight | 230.25 |
| - elemental analysis % | C=52.17%; H=7.88%; N=12.17% O=27.80% |
| - solubility in organic solvents | > 10 mg/ml in DMSO |
| - solubility in water | > 10 mg/ml |
| - melting point | / |
| - TLC | chloroform / methanol / H₂O/NH₃ (28 %) (80 : 25 : 2 : 1) Rf = 0.44 |

### Example 11 preparation of N,N'-bis (2-hydroxyethyl)-cromoglycholyldiamide (11)

5.74 g isobutylchloroformiate (42 mmol) in 50 ml DMF were slowly added drop by drop in 30 minutes to 10.25 g disodium cromoglycholate (20 mmol) in 150 ml anhydrous DMF under stirring at -10 °C. The mixture was maintained at -10 °C under stirring for 2 hours and then at 0 °C for 15 hours. 3.5 g ethanolamine were then slowly added drop by drop in 30 minutes. After 20 hours under stirring at 0 °C to the obtained suspension 300 ml of a NaCl saturated solution are added and the mixture obtained is extracted 3 times with 100 ml butanol, the extracted fractions are collected and evaporated to dryness. The residue is solubilized in 100 ml water, deionized with 20 ml Bio-Rad AG 501x8 resin and finally lyophilized. The reaction yield was about 71 %.

The physical-chemical characteristics of the N,N'-bis (2-hydroxyethyl)-cromoglycholyldiamide are the following:

| | |
|---|---|
| - physical state | white crystalline powder |
| - raw formula | C₂₇H₂₆N₂O₁₁ |
| - molecular weight | 554.52 |
| - elemental analysis % | C=58.48%; H=4.73%; N=5.05% O=31.74% |
| - solubility in organic solvents | > 10 mg/ml in DMSO |
| - solubility in water | > 10 mg/ml |
| - melting point | / |
| - TLC | chloroform / methanol / H₂O/NH₃ (28 %) (80 : 25 : 2 : 1) Rf = 0.24 |

### Example 12 preparation of N,N'-bis (2-hydroxyethyl)-aspartyldiamide (12)

2.67 g N-alpha-benzyloxycarbonylaspartic (10 mmol) were solubilized in 20 ml anhydrous methanol, 2 g of an anhydrous sulfonic resin Dowex 50x8 H⁺ was added to, and the obtained mixture was maintained under stirring at 50 °C for 24 hours. The resin was separated by filtration, the solution was evaporated to dryness and the residue treated with 1.34 ethanolamine at 45 °C for 20 hours.

The reaction mixture was solubilized in 50 ml ethanol/water 1:1 and eluted on a column containing 10 ml of the cationic ion exchange resin Dowex 50x8 generated under H⁺ form and cooled to 0 °C; the eluate was concentrated to dryness, resolubilized with 50 ml glacial acetic acid and hydrogenated for 20 hours in the presence of palladium black. The solution was evaporated to dryness, the residue resolubilized with 100 ml ethanol/water 1:1 end eluted on a column containing 10 ml of an anionic exchange resin Bowex 1x8 generated under OH⁻ form and cooled to 0 °C. The eluate was concentrated to about 10 ml and finally lyophilized. The reaction yield was about 70 %.

The physical-chemical characteristics of the N,N'-bis (2-hydroxyethyl)-aspartyldiamide are the following.

| | |
|---|---|
| - physical state | white crystalline powder |
| - raw formula | C₈H₁₇N₃O₄ |
| - molecular weight | 219.24 |
| - elemental analysis % | C=43.83%; H=7.82%; N=19.17% O=29.19% |
| - solubility in organic solvents | > 10 mg/ml in DMSO |
| - solubility in water | > 10 mg/ml |
| - melting point | / |
| - TLC | chloroform / methanol / H₂O/NH₃ (28 %) (88 : 25 : 2 : 1) Rf = 0.05 |

### Example 13 preparation of N,N' -bis (2-hydroxyethyl)-phthaloyldiamide (13)

A mixture of 1.94 g of dimethylphthalate (10 mmol) and 2.44 g ethanolamine (40 mmol) was introduced into a flask fitted with a reflux condenser and heated to 60 °C for 48 hours in an oil bath.

50 ml ethanol were added and the resulting solution was eluted on a column containing 25 ml of a sulfonic resin Dowex 50x8 generated under H⁺ form, the eluate was evaporated to dryness and the obtained oil was dried under high vacuum.

The reaction yield was about 78 %.

The physical-chemical characteristics of N,N'-bis (2-hydroxyethyl)-phthaloyldiamide produced are the following:

| | |
|---|---|
| - physical state | white crystalline powder |
| - raw formula | C₁₂H₁₆N₂O₄ |
| - molecular weight | 252.27 |
| - elemental analysis | C=57.13%; H=6.39%; N=11.11%; O=25.37%. |
| - solubility in organic solvents | > 10 mg/ml in ethanol |
| - solubility in water | > 10 mg/ml |
| - melting point | / |
| - TLC | chloroform/methanol/H₂O/NH₃ (28 %) (80 : 25 : 2 : 1) Rf = 0.36. |

### Example 14 preparation of N,N'-bis (2-hydroxyethyl)-trans,trans-muconoyldiamide (14)

1.42 g trans,trans-muconic acid (10 mmol) were solubilized into 20 ml anhydrous methanol and 2 g of anhydrous sulfonic resin Dowex 50x8 H⁺ were added to; the mixture was maintained under stirring at 60 °C for 48 hours. The resin was separated by filtration, the solution was evaporated to dryness and the residue treated with 1.34 g ethanolamine at 30 °C for 20 hours. The reaction mixture was directly crystallized from 50 ml isopropanol, the crystallized product was separated by filtration, washed 3 times with 10 ml of cool isopropanol and finally dried under high vacuum.

The reaction yield was about 74 %.

The physical-chemical characteristics of N,N'-bis (2-hydroxyethyl)-trans,trans-muconoyldiamide are the following:

| | |
|---|---|
| - physical state | white crystalline powder |
| - raw formula | C₁₀H₁₆N₂O₄ |
| - molecular weight | 228.25 |
| - elemental analysis | C=52.62%; H=7.07%; N=12.27%; O=28.04%. |
| - solubility in organic solvents | > 10 mg/ml in DMSO |
| - solubility in water | > 10 mg/ml |
| - melting point | 250 ° dec |
| - TLC | chloroform/methanol/H₂O/NH₃ (28 %) (88 : 25 : 2 : 1) Rf = 0.65. |

### Example 15 preparation of N,N'-bis-(2-hydroxyethyl)-glutaroyldiamide (15)

A mixture of 1.32 g glutaric acid (10 mmol) and 1.84 g ethanolamine (30 mmol) was introduced into a flask fitted with a reflux condenser and heated to 160 °C for 6 hours in an oil bath.

The reaction mixture was directly crystallized from 20 ml isopropanol, the crystallized product was separated by filtration, washed 3 times with 5 ml cool isopropanol and finally dried under high vacuum.

The reaction yield was about 72 %.

The physical-chemical characteristics of the N,N'-bis (2-hydroxyethyl)-glutaroyldiamide are the following:

| | |
|---|---|
| - physical state | white crystalline powder |
| - raw formula | C₉H₁₈N₂O₄ |
| - molecular weight | 218.26 |
| - elemental analysis | C=49.53%; H=8.31%; N=12.84%; O=29.32%. |
| - solubility in organic solvents | > 10 mg/ml in DMSO |
| - solubility in water | > 10 mg/ml |
| - melting point | 119 - 121 °C |
| - TLC | chloroform/methanol/H₂O/NH₃ (28 %) (80 : 25 : 2 : 1) Rf = 0.25. |

### Biologic activity against mast cells degranulation

In order to verify the specificity of the N-acyl derivatives according to the present invention to act as local autacoids when administered by exogenous route under conditions of degranulation induced by mast cells physiological stimuli, the following biological tests were carried out both in vivo and in vitro as described hereinbelow.

### In vivo biologic tests: topical and general application

- 2 weeks old Sprague Pawley rats, provided by Charles River from Calco, were locally treated by intradermal injection on the auricular pinna with the compounds in question at the dose of 0.5mg/kg, in a buffered aqueous solution at physiological pH.
   After 10 minutes a local administration, still by intradermal route, followed, of the substance P (10⁻⁴ M), able to induce a mast cells degranulation response.
   After 30' from the subtance P administration, the animals were sacrificed and the relative tissue samples (pinna) were taken, for an analysis of the morphological aspect of the mast cells residing in the connectival tissues after fixation and coloration with toluidine blue.
   The inhibition degree in mast cells degranulation in the tissues of the animals treated with the compounds under question, and compared with those of animals treated only with the physiological degranulation factor (substance P) was considered as a parameter for the biological activity.
   From the morphological analysis of the two conditions it resulted that whereas substance P induced a degranulation in the majority of mast cells, under pretreatment conditions with the compounds of the present invention a marked inhibition of this phenomenon was observed.

In table 1 the obtained results are reported.

**Table 1**

| Effects of the aminoalcohols N-acyl derivatives against the mast cells degranulation after intradermal administration at the dose of 0.5 mg/kg. | |
|---|---|
| tested substance | % of cells undergoing degranulation |
| solvent | 8 |
| substance P | 94 |
| N,N'-azelayldiethanolamide + subst. P | 30 |
| N,N'-fumaroyldiethanolamide + subst. P | 40 |

The pharmacologic activity of the compounds under question was also verified under conditions of a systemic subcutaneous administration.

In this case the animals were firstly treated with 20 mg/kg of the substances under question and after 30 minutes were exposed to the degranulation stimulus with the substance P (10⁻⁶ M) in the auricular pinna. Then we proceeded as previously described. In Tab.2 the obtained results are reported on an average of 500-800 cells.

**Tab.2**

| Effects of aminoalcohols N-acyl derivatives after subcutaneous administration of 20 mg/kg. | |
|---|---|
| tested substance | % of cells undergoing degranulation |
| solvent | 12 |
| substance P | 92 |
| (3) + Sub. P | 30 |
| (1) + Sub P | 28 |
| (4) + Sub. P | 32 |
| (2) + Sub. P | 32 |
| (5) + Sub. P | 45.5 |
| (6) + Sub. P | 33.6 |
| (9) + Sub. P | 32 |
| (7) + Sub. P | 35 |
| (8) + Sub. P | 31 |
| (10) + Sub. P | 38 |
| (11) + Sub. P | 28.6 |
| (12) + Sub. P | 30 |
| (13) + Sub. P | 35.5 |
| (14) + Sub. P | 40.2 |
| (15) + Sub. P | 30 |

### In vitro biologic test

Peritoneal mast cells of rat were taken according to the standard methodology described by Lagunoff (1975, Tech. Biochem. Biophys. Morphol. , 2, pp. 289-305).

The cells were then cultured in MEM which 10% fetal calf serum was added to and then incubated in a Haereus^{R} incubator for 30 minutes.

The derivatives under question at the concentration of 10⁻⁵ M were added to the incubation medium .

At the end of the incubation the physiological degranulation stimulus represented by the substance P (10⁻⁴) was added.

The cells were then centrifugated in order to remove the supernatant formed by the incubation medium and placed onto the slide after coloration with toluidine blue, for the analysis of the morphological aspect at the optical microcope.

Also under these conditions the parameter to be considered was the percentage of degranulated cells after stimulation with the Substance P.

The obtained results are indicated in Tab. 3

**Tab3**

| Effect of ethanolamine and diethanolamine N-acyl derivatives against in vitro mast cells. | |
|---|---|
| tested substance | % of cells undergoing degranulation |
| substance P | 96 |
| (3) + subst. P | 30 |
| (1) + subst. P | 35 |
| (4) + subst. P | 35 |
| (2) + subst. P | 30 |

These results demonstrate that the derivatives according to the present invention are able to modulate the degranulation processes induced by neuroimmunogenic stimuli, when they are administered by both local and general exogenous route, when the degranulation process induced by the Substance P is being carried out.

As the compounds according to the present invention are able to show their activity when they are both locally and systemically administered, the administration routes encompassed in the pathologies which can be treated according to the present invention are the dermal topical route, the intra- and trans-dermal route, the intraarticular route, the intracerebroventricular route, the corneal topical route, the intra- and retro-bulbar route, the vaginal route, the topical route on the gastric mucous membrane, the intranasal and the inhaling route, as well all the systemic administrations, and among them the oral route and the parenteral (intravenous, subcutaneous and intramuscular) route.

The necessary doses to have therapeutic effectiveness depend on the administration route and on the pathology seriousness. Furthermore other factors are to be considered connected with the patients' age, body weight and health general conditions. Anyway an acceptable therapeutic range is comprised between 0.1 mg/kg and 50 mg/kg and preferably between 0.5 and 20 mg/kg.

Relevant undesired side effects being unknown, further to the dosage, a therapeutic regimen has to be established based on medical criteria which take into account the acuity or the chronicity characteristics of the pathology.

Typically the therapeutic regimen may have chronicity features in connection with the different pathologies, with from 1 to 2 daily administrations for at least 4 weeks. In the case of specialistic applications as for example the intraarticular, the intracerebroventricular, the retrobulbar one, weekly administration may be foreseen for at least 4 weeks.

Furthermore, as these pathologies are characterized by new acute phases of the neuroimmunogenic symptomathologies, the aminoalcohol N-acyl derivatives according to the present invention can be advantageously used for a preventive action as well as for a therapeutic one.

Under these hazard conditions these compounds can be administered as dietetic integrating components - both to human beings and to animals.

The compositions containing as active ingredients the derivatives according to the present invention, comprise all the formulation suitable for the above amentioned administrations and the excipients may be those therapeutically or pharmacologically acceptable suitable for the same applications, as well new excipients able to improve the vehiculation of these compounds to the site of action.

In this connection also new vehiculation forms may be considered suitable, which can be obtained by bonding these compounds with specific markers of target tissues, which a preferential tropism, useful as specific vehiculation, exists for.

The preferred formulations for topical administration are the buffered solutions, collyria, gels, patches, lyophilized or granulated powders, suspensions, ovules, aerosols and sprays.

The topical administration of the compounds according to the present invention encompasses a dermocosmetic use, in particular for preventing skin diseases having autoimmune origin. For the oral systemic administration all the formulations result suitable in the form of dry powder such as granulates, tablets, dragees, perles and in the liquid form such as suspensions or oily perles.

The dietetic integrating components are preferably in the form of tablets or oily perles.

For the parenteral administration the preferred formulations are buffered aqueous solutions or oily suspensions also formed by a lyophilized product readily dispersable in the solvent at the moment of the administration.

The following examples of preferred pharmaceutical compositions are reported for illustrative but not limitative purposes.

### Example 1 : Lacquered tablets

Every tablet contains:

| | |
|---|---|
| (2) | 30 mg |
| O.P. lactose | 80 mg |
| O.P. maize starch | 75 mg |
| O.P. talc | 5 mg |
| O.P. magnesium stearate | 2 mg |
| hydroxypropylmethylcellulose | 2 mg |
| O.P. titanium bioxide | 1.2 mg |
| yellow iron oxide (E172) | 0.2 mg |

### Example 2 Jelly perles

Every perle contains

| | |
|---|---|
| (5) | 100 mg |
| O.P. peanuts oil | 100 mg |
| O.P. jelly | 52 mg |
| O.P glycerin | 16 mg |
| Erythrosin (E127) | 0.1 mg |

### Example 3: Lyophilized vials

Every lyophilized vial contains

| | |
|---|---|
| (4) | 50 mg |
| O.P. mannite | 57 mg |
| every vial contains : water for injectable formulations | 2 ml |

### Example 4: Dermatologic cream

100 g of cream contain:

| | |
|---|---|
| (3) | 50 mg |
| sorbitan monostearate | 500 mg |
| polyoxyethylensorbitan monostearate | 4.5 g |
| ethyl alcohol | 3 g |
| stearic acid | 3 g |
| paraffin oil | 10 g |
| 70% sorbitol | 6 g |
| methylester of p-benzoic acid | 0.2 g |
| propylester of p-benzoic acid | 0.05g |
| Water | q.s. to 100 g |

### Example 5 Ophtalmic ointment

| | |
|---|---|
| (1) | 5 g |
| mineral jelly | q.s. to 100 g |

The pharmaceutical compositions containing as the active principle the compounds of the present invention may find a valid therapeutic application both in human beings and animals, in all the pathologies having autoimmune origin, characterized by mast cells hyperstimulation and wherein it is necessary to modulate the degranulation process induced by neuroimmunogenic stimuli.

The application of these compositions results particularly useful in the following pathologies developing at the dermatologic level: psoriasis, epidermolysis bullosa, dermatomyositis, scleroderma, pemphigus and pemphigoid; at ophtalmic level: Sjogren's syndrome, uveites and uveoretinites; at the articular level, such as rheumatic arthritis, psoriasic arthritis, lupus erythematosus arthritis; at nervous level: multiple sclerosis, at the gastrointestinal level: the chronic inflammations of the gastrointestinal mucous membranes, at the systemic level such as systemic and discoid lupus erythematosus.

Other pathologic conditions wherein it is therapeutically useful to locally control the inflammatory processs are the chronic inflammatory pathologies (chronic arthritis, chronic heliumdermatitis, asthma, interstitial pulmonary fibrosis, allergic pathologies (allergic conjunctivitis and papillary giant conjunctivitis) and the dietetical alergies, cicatrization disorders (hypertrophic scars, ocular cicatricial pemphigoid, cheloid scars), the central and periferal nervous system pathologies, wherein it is ascertained that inflammatory/ edematous processes follow a primary injury having ischemic origin (peripheral neuropathies, cerebral trombosis, cranial trauma), as well as cardiac type pathologies, deriving from reperfusion phenomena as a consequence of ischemic insults.

Furthermore, the administration of the compounds described in the present invention is useful in some particular veterinary pathologies such as neurogenic inflammation (i.e. in intravertebral disk disease of dog or traumatic lesion); ophtalmic inflammatory pathology (i.e. Keratoconjunctivitis sicca), articular or connective inflammation (i.e. laminitis and arthritis), respiratory inflammatory pathologies and in general reaction of inflammatory origin including allergy.

## Claims

1. Use of a N-acyl derivative of an aminoalcohol of formula: 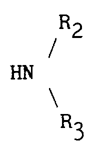 wherein R₂ is an alcoholic residue selected from a C₁-C₂₀ linear or branched hydroxyalkyl optionally substituted in the aliphatic chain with one or more aryl groups and a hydroxyaryl optionally substituted with one or more linear or branched alkyl radicals of from 1 to 20 carbon atoms, and R₃ is H or is = R₂ with a polycarboxylic acid as the active principle for the preparation of a pharmaceutical composition for the preventive and the therapeutical treatment of human and animal pathologies undergoing mast cell degranulation as a consequence of neurogenic and/or immunogenic hyperstimulation.

2. The use according to claim 1, characterized in that said aminoalcohol is selected from the group consisting of ethanolamine, diethanolamine, 2-hydroxypropylamine, di-(2hydroxypropyl)amine.

3. The use according to claim 1 characterized in that when the aminoalcohol is 2-hydroxypropylamine or di-(2hydroxypropyl)amine, said N-acylderivative is an optical isomer or a raceme.

4. The use according to claim 1 characterized in that said polycarboxylic acid is a saturated or an unsaturated aliphatic dicarboxylic acid, optionally substituted with a hydroxy ar an aminic group.

5. The use according to claim 4 characterized in that said saturated or unsaturated dicarboxylic acid is selected from the group consisting of: oxalic, fumaric, azelaic, succinic, traumatic , glutaric acid, the muconic acids, and their hydroxy- or amino-substituted homologues.

6. The use according to claim 1 characterized in that said polycarboxylic acid is an aromatic, heteroaromatic or heterocyclic dicarboxylic acid.

7. The use according to claim 6 characterized in that said aromatic, heterocyclic or heteroaromatic dicarboxylic acid is selected from the group consisting of phthalic, 1,4 dihydroxy-2,6-dimethyl-4-(2-nitrophenyl)-3,5-pyridin-dicarboxylic acid, 4,6-dioxo-1-ethyl-10-propyl-4H,6H,-pyran-[3,2g]-quinolin-2,8-dicarboxylic cromoglycholic acid and other biologically acceptable acids.

8. The use according to claim 1 characterized in that said polycarboxylic acid is a tricarboxylic acid consisting of citric acid.

9. The use according to claim 1 wherein said human pathologies are multiple sclerosis, psoriasis, epidermolysis bullosa, dermatomyositis, scleroderma, pemphigus and pemphigoid; Sjogren's syndrome, autoimmune uveites and uveoretinites; rheumatic arthritis, psoriatic arthritis, lupus erythematosus arthritis, chronic inflammations of the gastrointestinal mucous membranes, systemic and discoid lupus erythematosus, chronic arthritis, chronic heliumdermatitis, asthma, interstitial pulmonary fibrosis, allergic conjunctivitis and papillary giant conjunctivitis alergies, hypertrophic scars, ocular cicatricial pemphigoid, cheloid scars, peripheral neuropathies, cerebral trombosis, cranial trauma, and cardiac type pathologies, deriving from reperfusion phenomena as a consequence of ischemic insults, and the animal pathologies are neurogenic inflammations selected from intravertebral disk disease, articular or connective pathologies, respiratory pathologies, laminitis, eye pathologies selected from Keratoconjunctivitis sicca and finally inflammatory allergy manifestations selected from food allergy.

10. The use according to claim 1 characterized in that said pharmaceutical compositions are suitable for the topical administration and are selected from the class consisting of: buffered solutions, collyria, gels, patches, lyophylized or granulated powders, suspensions, ovules, aerosols and sprays.

11. The use according to claim 9 characterized in that said topical compositions are dermocosmetic compositions for preventing human skin diseases having autoimmune origin.

12. The use according to claim 1 characterized in that said pharmaceutical compositions are suitable for the oral systemic administration and are in the form of dry powders and are selected from the groups consisting of: granulates, tablets, dragees, perles or in a liquid form selected from the group consisting of suspensions and oily perles.

13. The use according to claim 1 characterized in that said pahrmaceutical compositions are dietetic integrating components for human beings and animals.

14. The use according to claim 1 characterized in that said pharmaceutical compositions are suitable for the parenteral administration in the form of buffered aqueous solutions or oily suspensions also formed by a lyophilized product readily dispersable in the solvent at the moment of the administration.
